# EUROPÄISCHE PATENTSCHRIFT

(11) **EP 0 370 399 B1**
(45) Veröffentlichungstag und Bekanntmachung des Hinweises auf die Patenterteilung: **21.06.1995**
(21) Anmeldenummer: 89121277.1
(22) Anmeldetag: 17.11.1989
(51) Int. Cl.: C07K 5/08, C12P 21/02

(54) **Verfahren zur Herstellung von Tripeptiden**
Process for the preparation of tripeptides
Procédé de préparation de tripeptides

(30) Priorität: 22.11.1988 DE 3839379
(43) Veröffentlichungstag der Anmeldung: 30.05.1990
(73) Patentinhaber: HOECHST AKTIENGESELLSCHAFT, 65926 Frankfurt am Main (DE)
(72) Erfinder: Flemming, Hans-Wolfram, Dr., D-6390 Usingen (DE); Rukwied, Manfred, Dr., D-6233 Kelkheim(Taunus) (DE); Schmidt, Manfred, D-6292 Weilmünster (DE)

(56) Entgegenhaltungen:
- EP-A- 0 156 280
- EP-A- 0 178 553
- WO-A-79/00602
- US-A- 4 293 648
- BIOMEDICA BIOCHIMICA ACTA, Band 44, Nr. 2, 1985, Seiten 175-183, Akademie-Verlag, Berlin, DE; U. ROTHE et al.: "Kinetische Untersuchungen der Hydrolysevon Alaninpeptidestern und -p-Nitraniliden durch Thermitase, einerthermostabilen Serinprotease aus Thermoactinomyces vulgaris:Sekundärspezifität, Temperatur- und Lösungsmitteleinfluss"
- FEDERATION PROCEEDINGS, Band 28, Nr. 2, März-April 1969, Seite 657, Zusammenfassung Nr. 2224, Federation of American Societies for Experimental Biologx; A.F. ELEN: "Enzymatic splitting of 1-alanyl-D-alamine from UDP-acetyl-muramyl-fentapeptide catalyzed by a soluble enzyme from Bacillus subtilis"

## Beschreibung

Tripeptide sind wichtige Zwischenprodukte bei der Synthese bioaktiver Peptide, wie zum Beispiel des Hypothalamus-Hormons Gonadorelin und dessen Analoga. Zu diesem Zweck müssen die Tripeptide in möglichst einfacher Weise einerseits in guten Ausbeuten und andererseits in hoher Reinheit zugänglich sein. Die bisher bekannten Verfahren zur Herstellung von Tripeptiden erfüllen diese Anforderungen nicht in optimaler Weise und sind mit teilweise schweren Nachteilen behaftet. So sind beispielsweise auch die nach dem in der EP-A 156 280 beschriebenen Verfahren hergestellten Produkte mit Nebenprodukten verunreinigt, die sich in den weiteren Syntheseschritten nachteilig bemerkbar machen. Aufgabe der vorliegenden Erfindung ist es deshalb, ein Verfahren zur Herstellung von Tripeptiden bereitzustellen, das die genannten Nachteile nicht aufweist und in einfacher Weise Produkte hoher Reinheit in guten Ausbeuten liefert.

Demgemäß betrifft die Erfindung ein Verfahren zur Herstellung von Tripeptiden der allgemeinen Formel I

U - A - B - C - OH I

worin
- U: Wasserstoff oder eine Urethanschutzgruppe
- A: eine natürliche α-Aminosäure oder Abkömmlinge davon
- B: eine natürliche α-Aminosäure oder Abkömmlinge davon und
- C: eine aromatische α-Aminosäure bedeutet,
das dadurch gekennzeichnet ist, daß
eine Verbindung der allgemeinen Formel II

U′ - B - OH II

worin U′ eine hydrogenolytisch abspaltbare Urethanschutzgruppe ist und B die obengenannte Bedeutung hat mit einer Verbindung der allgemeinen Formel III

H - C - OR III

worin R für Alkyl mit 1 bis 4 Kohlenstoffatomen steht und C die obengenannte Bedeutung hat in Gegenwart von Propylphosphonsäureanhydrid umgesetzt, die Schutzgruppe U′ hydrogenolytisch abgespaltet, die so erhaltene Verbindung der allgemeinen Formel IV

H - B - C - OR IV

mit einer Verbindung der allgemeinen Formel V

U - A - OH V

in Gegenwart von Propylphosphonsäureanhydrid umgesetzt und schließlich R enzymatisch abgespalten wird.

Die für U stehenden Urethanschutzgruppen sind bevorzugt die in der Peptidchemie üblichen Urethanschutzgruppen wie sie beispielsweise in Kontakte Merck 3/79, Seite 14, beschrieben sind.
Besonders bevorzugt sind die Benzyloxycarbonyl- und die tert.-Butyloxycarbonyl-Gruppe.
Eine hydrogenolytisch abspaltbare Urethanschutzgruppe U′ ist bevorzugt die Benzyloxycarbonyl-Gruppe.

Für A und/oder B stehende natürliche α-Aminosäuren oder deren Abkömmlinge sind bevorzugt Gly, Ala, Ser, Thr, Val, Leu, Ile, Glu, Gln, p-Glu, Tyr, Phe, Trp und His. Besonders bevorzugt sind Ser, Thr, Trp und Phe.

Eine für C stehende aromatische α-Aminosäure ist bevorzugt Tyr oder Phe.

In der allgemeinen Formel IV bedeutet R bevorzugt Methyl.

Ganz besonders bevorzugt ist ein Verfahren, in dem U und U′ Benzyloxycarbonyl, A Trp, B Ser, C Tyr und R Methyl bedeuten.

Die Knüpfung einer Peptidbindung in Gegenwart von Propylphosphonsäureanhydrid ist als PPA-Methode bekannt (Angew. Chem. Int. Ed. 19, 133 (1980)).
Bevorzugt wird diese Umsetzung in polaren Lösungsmitteln wie beispielsweise Dimethylacetamid, Dimethylformamid, Dimethylsulfoxid, Phosphorsäure-tris-(di-methylamid), N-Methylpyrrolidon oder Wasser ausgeführt. Aber auch Chloroform, Methylenchlorid oder Essigester kommen zum Einsatz.
Vorteilhafterweise können auch Mischungen der genannten Lösungsmittel mit Wasser verwendet werden. Besonders bevorzugt ist ein Essigester/Wasser-Gemisch. Die Synthese kann zwischen -10 °C und Raumtemperatur durchgeführt werden. Vorzugsweise beginnt man bei ca. 0 °C und erhöht später auf Raumtemperatur.

Die hydrogenolytische Abspaltung der U′-Schutzgruppe geschieht vorteilhaft in bekannter Weise mit Wasserstoff am Pd/C-Kontakt.

Die enzymatischer Esterolyse im letzten Reaktionsschritt wird bevorzugt mit Trypsin und/oder α-Chymotrypsin ausgeführt (Hoppe-Seylers Zeitschrift f. physiol. Chemie, 336, 248 (1964)). Besonders bevorzugt ist Trypsin. Gegebenenfalls werden auch Enzyme verwendet, die nach bekannten Methoden an einen Träger immobilisiert sind, wie zum Beispiel in der EP-A 178 553 beschrieben. Die Enzyme werden dabei vorteilhafterweise in Mengen von 0,01 bis 20 Gew.-% bezogen auf die Substratmenge eingesetzt. Besonders bevorzugt ist eine Enzymmenge von 2 Gew.-%

Als Lösungsmittel können beispielsweise Wasser, Dimethylformamid, Methanol, Ethanol, Isopropanol, Butanol, Ethylacetat, Butylacetat, Toluol oder Methylenchlorid eingesetzt werden.
Bevorzugt ist ein Essigester/Wassergemisch.
Die Temperaturen liegen vorteilhafterweise zwischen 0 und 60 °C. Bevorzugt ist ein Temperaturenbereich von 20 bis 35 °C. Der pH-Wert des Reaktionsmediums bewegt sich bevorzugt zwischen 4 und 10, besonders bevorzugt zwischen 4 und 8

Das erfindungsgemäße Verfahren kann so ausgeführt werden, daß jede Zwischenstufe isoliert wird. Bevorzugt wird aber im Eintopfverfahren, beziehungsweise ohne Isolierung der Zwischenstufen gearbeitet.
Die Ausgangsverbindungen der allgemeinen Formeln II, III und V sind bekannt und können nach den üblichen Methoden erhalten werden.

Das erfindungsgemäße Verfahren liefert überraschenderweise Produkte hoher chemischer und optischer Reinheit, die ohne Probleme in weitere Synthesen eingesetzt werden können. Die Ausbeuten sind ebenfalls hervorragend und liegen zwischen 40 und 50 % bezogen auf die eingesetzte Menge der Verbindung der allgemeinen Formel III.

Es ist als besonders überraschend anzusehen, daß das erfindungsgemäBe Verfahren, sowohl hinsichtlich Reinheit als auch Ausbeute, dem nur dreistufigen Verfahren der EP-A 156 280 deutlich überlegen ist.

### Beispiel

### Z-Trp-Ser-Tyr-OH

a) In einer 2 l Rührapparatur werden 350 ml Wasser vorgelegt, 47,8 g (0,200 mol) Z-Ser-OH, 46,4 g (0,200 mol) H-Tyr-OMexHCl und 150 g Natriumchlorid eingetragen. Weiterhin werden 700 ml Ethylacetat zugegeben und nachdem sich alles gelöst hat, wird der pH-Wert des Gemisches durch Zugabe von ca. 25 ml N-Ethylmorpholin auf 5,0 gestellt. Während der Zugabe von ca. 220 ml (0,42 mol) PPA-Lösung (w(PPA) in % = 50) in etwa 30 Minuten bei maximal 30 °C (am Ende etwas kühlen) werden über eine pH-Stat-Pumpe bei pH 5,0 ca. 110 ml (0,86 mol) N-Ethylmorpholin zugegeben. Die PPA-Zugabe ist beendet, wenn sich im Reaktionsgemisch ein Niederschlag bildet. Durch erneute Zugabe von 350 ml Wasser wird der Niederschlag wieder gelöst. Nach Abtrennung der Wasserphase in einem Scheidetrichter wird die Esterphase mit 700 ml Kaliumhydrogensulfatlösung (w(KHSO₄) in % = 10) 700 ml Natriumhydrogencarbonatlösung (w(NaHCO₃) in % = 5) gewaschen. Die Wasserphase der Reaktion und die Waschphasen werden verworfen.
b) Ca. 700 ml Esterphase der 1. Kupplung, 200 ml Wasser und 3,3 g Palladium auf Kohle w(Pd) in % = 2,5 werden in einer 2 l Rührapparatur vorgelegt und bei 25-30 °C mit einem Wasserstoffstrom beaufschlagt. Während der Reaktion wird der pH-Wert mit einer pH-Stat-Pumpe und Zugabe von ca. 160 ml (0,16 mol) Salzsäure c(HCl) = 1 mol/l auf 4,0 gehalten. Nach beendeter Reaktion, wenn keine Salzsäure mehr verbraucht wird, (ca. 30 Minuten) wird das Reaktionsgemisch über eine Nutsche abgesaugt und im Scheidetrichter die Wasserphase von der Esterphase abgetrennt. Die Esterphase wird verworfen.
c) Ca. 430 ml Wasserphase der Hydrogenolyse, 700 ml Ethylacetat werden in einer 2 l Rührapparatur vorgelegt und 50,7 g (0,15 mol) Z-Trp-OH und 125 g Natriumchlorid zugegeben. Nachdem sich alles gelöst hat, wird ca. 19 ml N-Ethylmorpholin der pH-Wert auf 5,0 gestellt. Währen der Zugabe von ca. 220 ml (0,42 mol) PPA-Lösung (w(PPA) in % = 50) in etwa 30 Minuten bei maximal 30 °C (am Ende etwas kühlen) werden aber eine pH-Stat-Pumpe bei pH 5,0 ca. 110 ml (0,86 mol) N-Ethylmorpholin zugegeben. Die PPA-Zugabe ist beendet, wenn sich im Reaktionsgemisch ein Niederschlag bildet. Durch erneute Zugabe von 350 ml Wasser wird der Niederschlag wieder gelöst. Nach Abtrennen der Wasserphase in einem Scheidetrichter wird die Esterphase mit 700 ml Kaliumhydrogensulfatlösung (w(KHSO₄) in % = 10) und bis zur vollständigen Entfernung (nach DC-Analyse) von Z-Trp-OH mehrmals mit je 700 ml Natriumhydrogencarbonatlösung (w(NaHCO₃) in % = 5) gewaschen. Die Wasserphase der Reaktion und die Waschphasen werden verworfen.
d) Ca. 700 ml Esterphase der 2. Kupplung und 700 ml Wasser werden in einer 2 l Rührapparatur vorgelegt und auf 35-40 °C erwärmt und zunächst mit 1 g Trypsin versetzt. Während der sofort eingesetzenden Reaktion wird der pH.Wert mit ca. 110 ml (0,11 mol) Natronlauge (c(NaOH) = 1 mol/l) konstant auf pH 7,0 gehalten. Die Reaktion dauert etwa 7 Stunden und wird zwischendurch immer wieder durch weitere Zugabe von 0,5 g Trypsin beschleunigt. Sie ist beendet, wenn die Trypsinzugabe nur noch eine geringe Beschleunigung der Natronlaugeaufnahme bewirkt oder nach DC-Analyse kaum noch Ausgangsprodukt aufweist. Die Reaktionslösung wird über eine Nutsche geklärt und im Scheidetrichter die Esterphase von der Wasserphase abgetrennt. Die Esterphase wird verworfen.
   Die Wasserphase wird zunächst zweimal bei pH 5,8-6,0 durch Zugabe und Auflösen 4,0 g Kaliumdihydrogenphsophat mit je 700 ml Ethylacetat ausgeschüttelt. Die Esterphasen werden verworfen. Die Wasserphase wird dann anschließend dreimal bei pH 5,0, eingestellt durch Zugabe von ca. 5 ml Eisessig, mit je 700 ml Ethylacetat ausgeschüttelt. Die Wasserphase wird verworfen. Die Esterphasen enthalten das Tripeptid, welches beim Eindampfen zur Trockene im Vakuum in lockerer kristalliner Form zurückbleibt. Das Produkt wird im Vakuumtrockenschrank bei 40 °C getrocknet.

- Auswaage:: 51,2 g
- Ausbeute:: 42,0 % bez. auf H-Tyr-OMexHCl
- Reinheit:: 98,2 % (bestimmt mit HPLC LiChrosorb Si 60/Peptid-Puffer)

### Vergleichsbeispiel

Z-Trp-Ser-Tyr-OH wurde nach dem in der EP-A 156 280 angegebenen Verfahren hergestellt.
- Ausbeute:: 30 %
- Reinheit:: 78,8 % (bestimmt mit HPLC LiChrosorb Si 60/Peptid-Puffer)

## Patentansprüche

1. Verfahren zur Herstellung von Tripeptiden der allgemeinen Formel I
U - A - B - C - OH I
worin
U Wasserstoff oder eine Urethanschutzgruppe
A eine natürliche α-Aminosäure oder Abkömmlinge davon
B eine natürliche α-Aminosäure oder Abkömmlinge davon und
C eine aromatische α-Aminosäure bedeutet,
dadurch gekennzeichnet, daß eine Verbindung der allgemeinen Formel II
U′ - B - OH II
worin U′ eine hydrogenolytisch abspaltbare Urethanschutzgruppe ist und B die obengenannte Bedeutung hat mit einer Verbindung der allgemeinen Formel III
H - C - OR III
worin R für Alkyl mit 1 bis 4 Kohlenstoffatomen steht und C die obengenannte Bedeutung hat in Gegenwart von Propylphosphonsäureanhydrid umgesetzt, die Schutzgruppe U′ hydrogenolytisch abgespaltet, die so erhaltene Verbindung der allgemeinen Formel IV
H - B - C - OR IV
mit einer Verbindung der allgemeinen Formel V
U - A - OH V
in Gegenwart von Propylphosphonsäureanhydrid umgesetzt und schließlich R enzymatisch abgespalten wird.

2. Verfahren gemäB Anspruch 1, dadurch gekennzeichnet, daß U Benzyloxycarbonyl oder tert.-Butyloxycarbonyl bedeutet.

3. Verfahren gemäß anspruch 1 und/oder 2, dadurch gekennzeichnet, daß A und/oder B Gly, Ala, Ser, Thr, Val, Leu, Ile, Glu, Gln, p-Glu, Tyr, Phe, Trp oder His bedeuten.

4. Verfahren gemäß einem oder mehreren der Ansprüche 1 bis 3, dadurch gekennzeichnet, daß A und/oder B Ser, Thr, Trp oder Phe bedeuten.

5. Verfahren gemäß einem oder mehreren der Ansprüche 1 bis 4, dadurch gekennzeichnet, daß C Tyr oder Phe bedeutet.

6. Verfahren gemäß einem oder mehreren der Ansprüche 1 bis 5, dadurch gekennzeichnet, daß U′ Benzyloxycarbonyl bedeutet.

7. Verfahren gemäß einem oder mehreren der Ansprüche 1 bis 6, dadurch gekennzeichnet, daß R Methyl bedeutet.

8. Verfahren gemäß einem oder mehreren der Ansprüche 1 bis 7, dadurch gekennzeichnet, daß die Umsetzungen in Gegenwart von Propylphosphonsäureanhydrid in einem Essigester/Wasser-Gemisch ausgeführt werden.

9. Verfahren gemäß einem oder mehreren der Ansprüche 1 bis 8, dadurch gekennzeichnet, daß die enzymatische Esterolyse mit Trypsin und/oder α-Chymotrypsin ausgeführt wird.

10. Verfahren gemäß einem oder mehreren der Ansprüche 1 bis 9, dadurch gekennzeichnet, daß U und U′ Benzyloxycarbonyl, A Trp, B Ser, C Tyr und R Methyl bedeuten.

## Claims

1. A process for the preparation of tripeptides of the formula I
U - A - B - C - OH I
in which
U denotes hydrogen or a urethane protective group
A denotes a natural α-amino acid or derivatives thereof
B denotes a natural α-amino acid or derivatives thereof and
C denotes an aromatic α-amino acid,
which comprises reacting a compound of the formula II
U' - B - OH II
in which U' is a urethane protective group which can be eliminated by hydrogenolysis, and B has the abovementioned meaning, with a compound of the formula III
H - C - OR III
in which R represents alkyl having 1 to 4 carbon atoms, and C has the abovementioned meaning, in the presence of propylphosphonic anhydride, eliminating the protective group U' by hydrogenolysis, reacting the resulting compound of the formula IV
H - B - C - OR IV
with a compound of the formula V
U - A - OH V
in the presence of propylphosphonic anhydride, and finally eliminating R enzymatically.

2. The process as claimed in claim 1, wherein U denotes benzyloxycarbonyl or tert.-butyloxycarbonyl.

3. The process as claimed in claim 1 and/or 2, wherein A and/or B denote Gly, Ala, Ser, Thr, Val, Leu, Ile, Glu, Gln, p-Glu, Tyr, Phe, Trp or His.

4. The process as claimed in one or more of claims 1 to 3, wherein A and/or B denote Ser, Thr, Trp or Phe.

5. The process as claimed in one or more of claims 1 to 4, wherein C denotes Tyr or Phe.

6. The process as claimed in one or more of claims 1 to 5, wherein U' denotes benzyloxycarbonyl.

7. The process as claimed in one or more of claims 1 to 6, wherein R denotes methyl.

8. The process as claimed in one or more of claims 1 to 7, wherein the reactions are carried out in the presence of propylphosphonic anhydride in an ethyl acetate/water mixture.

9. The process as claimed in one or more of claims 1 to 8, wherein the enzymatic esterolysis is carried out with trypsin and/or α-chymotrypsin.

10. The process as claimed in one or more of claims 1 to 9, wherein U and U' denote benzyloxycarbonyl, A denotes Trp, B denotes Ser, C denotes Tyr and R denotes methyl.

## Revendications

1. Procédé de préparation de tripeptides de formule générale I,
U - A - B - C - OH (I)
dans laquelle
U représente un atome d'hydrogène ou un groupe protecteur formant une fonction uréthane,
A représente un acide α-aminé naturel ou un dérivé de cet acide,
B représente un acide α-aminé naturel ou un dérivé de cet acide, et
C représente un acide α-aminé aromatique,
qui est caractérisé en ce que qu'on fait réagir, en présence d'anhydride d'acide propylphosphonique, un composé de formule générale II,
U' - B - OH (II)
dans laquelle U' représente un groupe protecteur formant une fonction uréthane séparable par hydrogénolyse et B a la définition indiquée
ci-dessus, avec un composé de formule générale III,
H - C - OR (III)
dans laquelle R représente un groupe alkyle en C₁₋₄ et C a la définition indiquée ci-dessus, on élimine par hydrogénolyse le groupe protecteur U', on fait réagir, en présence d'anhydride d'acide propylphosphonique, le composé de formule générale IV ainsi obtenu,
H - B - C - OR (IV)
avec un composé de formule générale V,
U - A - OH (V)
et on élimine finalement R par clivage enzymatique.

2. Procédé selon la revendication 1, caractérisé en ce que U désigne le groupe benzyloxycarbonyle ou le groupe tert-butoxycarbonyle.

3. Procédé selon la revendication 1 et/ou 2, caractérisé en ce que A et/ou B désignent Gly, Ala, Ser, Thr, Val, Leu, Ile, Glu, Gln, p-Glu, Tyr, Phe, Trp ou His.

4. Procédé selon une ou plusieurs des revendications 1 à 3, caractérisé en ce que A et/ou B désignant Ser, Thr, Trp ou Phe.

5. Procédé selon une ou plusieurs des revendications 1 à 4, caractérisé an ce que C désigne Tyr ou Phe.

6. Procédé selon une ou plusieurs des revendications 1 à 5, caractérisé en ce que U' désigne le groupe benzyloxycarbonyle.

7. Procédé selon une ou plusieurs des revendications 1 à 6, caractérisé en ce que R désigne le groupe méthyle.

8. Procédé selon une ou plusieurs des revendications 1 à 7, caractérisé en ce que les réactions réalisées en présence d'anhydride d'acide propylphosphonique ont lieu dans un mélange acétate d'éthyle/eau.

9. Procédé selon une ou plusieurs des revendications 1 à 8, caractérisé en ce que l'estérolyse enzymatique est effectuée avec de la trypsine et/ou de l'α-chymotrypsine.

10. Procédé selon une ou plusieurs des revendications 1 à 9, caractérisé en ce que U et U' désignent le groupe benzyloxycarbonyle, A désigne le groupe Trp, B désigne le groupe Ser, C désigne le groupe Tyr et R désigne le groupe méthyle.
